(19) Europäisches Patentamt
European Patent Office
Office européen des brevets

(11) Veröffentlichungsnummer: **0 176 999**
**A2**

(12) # EUROPÄISCHE PATENTANMELDUNG

(21) Anmeldenummer: 85112356.2

(22) Anmeldetag: 30.09.85

(51) Int. Cl.⁴: **C 07 K 5/06**
A 61 K 37/02, A 61 K 37/64

(30) Priorität: 05.10.84 DE 3436569

(43) Veröffentlichungstag der Anmeldung:
09.04.86 Patentblatt 86/15

(84) Benannte Vertragsstaaten:
AT BE CH DE FR GB IT LI LU NL SE

(71) Anmelder: BOEHRINGER INGELHEIM KG
D-6507 Ingelheim am Rhein(DE)

(84) Benannte Vertragsstaaten:
BE CH DE FR IT LI LU NL SE AT

(71) Anmelder: Boehringer Ingelheim International G.m.b.H
D-6507 Ingelheim am Rhein(DE)

(84) Benannte Vertragsstaaten:
GB

(72) Erfinder: Roos, Otto, Dr.
Elsheimer Strasse 36
D-6501 Schwabenheim(DE)

(72) Erfinder: Schnorrenberg, Gerd, Dr.
Fichtenweg 13
D-6507 Ingelheim/Rhein(DE)

(72) Erfinder: Lösel, Walter
Im Herzenacker 26
D-6535 Gau-Alfesheim(DE)

(72) Erfinder: Wiedemann, Ingrid, Dr.
Ernst-Ludwig-Strasse 61
D-6535 Gau-Algesheim(DE)

(72) Erfinder: Gaida, Wolfram, Dr.
Paul-Clemen-Strasse 14
D-6507 Ingelheim/Rhein(DE)

(72) Erfinder: Hoefke, Wolfgang, Dr.
Rosselstrasse 27
D-6200 Wiesbaden(DE)

(54) **Aminosäurederivate, Verfahren zu ihrer Herstellung und pharmazeutische Zusammensetzungen.**

(57) Die Erfindung betrifft neue Aminosäurederivate der allgemeinen Formel

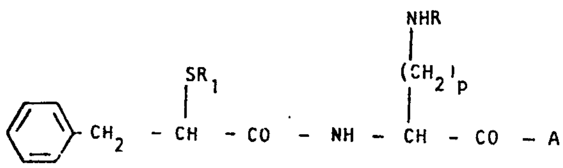

(I)

sowie ihre Salze.

Gegenstand der Erfindung sind ferner Verfahren zu ihrer Herstellung, pharmazeutische Zusammensetzungen und die Anwendung als Arzneimittel.

In Formel I bedeuten:

$R_1$ H, Acetyl, Benzoyl, Pivaloyl oder den Rest

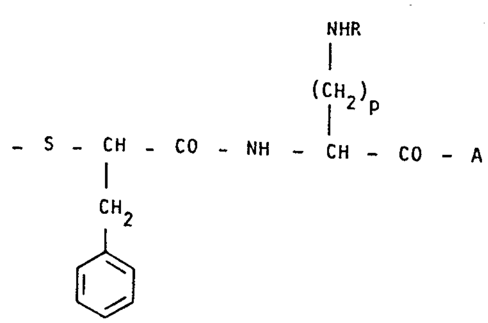
;

./...

EP 0 176 999 A2

P eine der Zahlen 1 – 4;
A den Rest einer der α-Aminosäuren

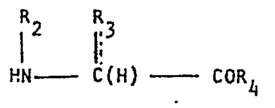

oder

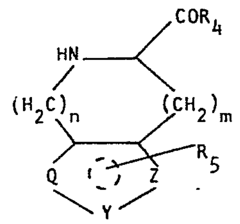

$R_2$ Wasserstoff, $C_1$-$C_4$-Alkyl, gerade oder verzweigt, $C_5$-$C_7$-Cycloalkyl, wobei ein Phenylring ankondensiert sein kann, Phenyl, Phenyl-$C_1$-$C_4$-alkyl, einen Hetero-$C_1$-$C_4$-alkylrest, wobei der Heterocyclus aus einem 5- oder 6-Ring mit 1 – 2 der Heteroatome O, S oder N besteht,

$R_3$ Wasserstoff, $C_1$-$C_4$-Alkyl, gerade oder verzweigt, Phenyl, Phenyl-$C_1$-$C_4$-alkyl oder einen Hetero-$C_1$-$C_4$-alkylrest, wobei der Heterocyclus aus einem 5- oder 6-Ring mit 1 – 2 der Heteroatome O, S oder N besteht, oder

$R_2$ und $R_3$ zusammen bilden mit dem N- und dem C-Atom einen 5-, 6- oder 7-gliedrigen Ring, der gesättigt sein oder eine Doppelbindung enthalten kann, oder einen 4-, 5- oder 6-gliedrigen Ring, der ein oder zwei weitere der Heteroatome O, S oder N enthält;

$R_4$ OH, $C_1$-$C_4$-ω-Hydroxyalkyl, $C_1$-$C_4$-Alkoxy, Phenyl-$C_1$-$C_4$-alkoxy, $C_1$-$C_4$-Akylamino, $(C_1$-$C_4)_2$-Dialkylamino, eine der Gruppen

oder eine α-Aminosäure, die peptidartig mit der CO-Gruppe des Moleküls verknüpft ist;

$R_5$ $C_1$-$C_4$-Alkyl, $C_1$-$C_3$-Alkenyl, $C_1$-$C_3$-Alkinyl, gerade oder verzweigt, Hydroxy, Nitro, Amino, $C_1$-$C_4$-Alkoxy, Mercapto, $C_1$-$C_4$-Alkylthio, Hydroxy-$C_1$-$C_4$-alkyl, Mercapto-$C_1$-$C_4$-alkyl, F, Cl, Br, Amino-$C_1$-$C_4$-alkyl, Sulfonamido, Methylendioxy, Fluor-$C_1$-$C_4$-alkyl, Chlor-$C_1$-$C_4$-alkyl, Brom-$C_1$-$C_4$-alkyl, Cyano oder Trifluormethyl;

$R_6$ Wasserstoff oder Methyl;

$R_7$ $C_1$-$C_4$-Alkyl, gerade oder verzweigt, wobei der Alkylrest durch F, Cl, Br, $CF_3$, Phenyl oder Pyridyl substituiert sein kann;

X, Y und Z O, S, $NR_8$, $CR_9$, $CHR_9$,

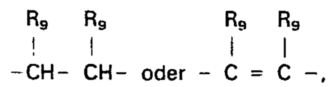

mit der Maßgabe, daß nur einer der Reste X, Y und Z O, S

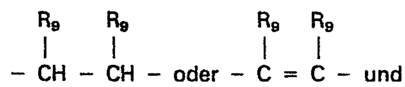

einer oder zwei der Reste X, Y und Z $NR_8$ bedeuten können;

$R_8$ Wasserstoff oder $C_1$-$C_4$-Alkyl, gerade oder verzweigt;

$R_9$ Wasserstoff, oder zusammen mit einem vicinal stehenden Rest $R_9$ einen Phenylring oder, für m und n = 1, dessen Dihydroform mit der Doppelbindung in Konjugation zur C-terminalen Carboxygruppe und

m und n jeweils 0, 1 oder 2, wobei die Summe aus m und n 1 oder 2 ist.

Die neuen Verbindungen besitzen eine starke Angiotensin-I-Converting-Enzym-Hemmung und sollen als Hypotensiva Verwendung finden.

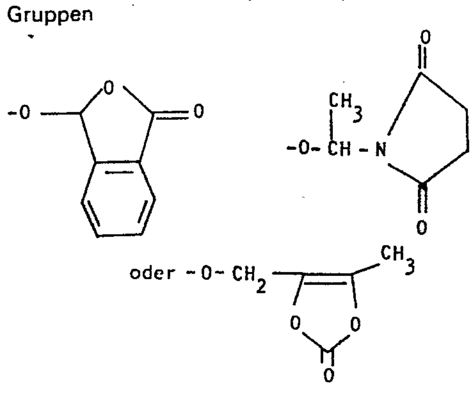

oder

oder 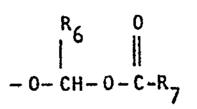

Die Erfindung betrifft neue Aminosäurederivate der allgemeinen Formel

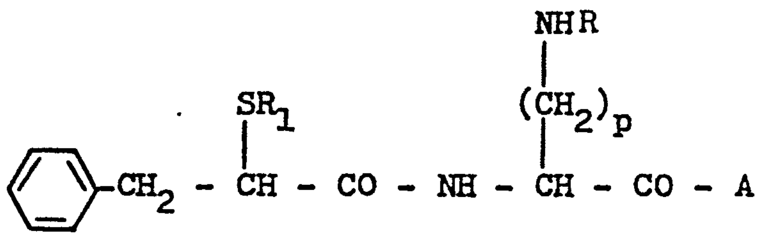 (I)

sowie ihre Salze.
Gegenstand der Erfindung sind ferner Verfahren zu ihrer Herstellung, pharmazeutische Zusammensetzungen und die Anwendung als Arzneimittel.

In Formel I bedeuten:

R H, Acetyl, Benzoyl, Pivaloyl oder t. butoxycarbonyl;

$R_1$ H, Acetyl, Benzoyl, Pivaloyl oder den Rest

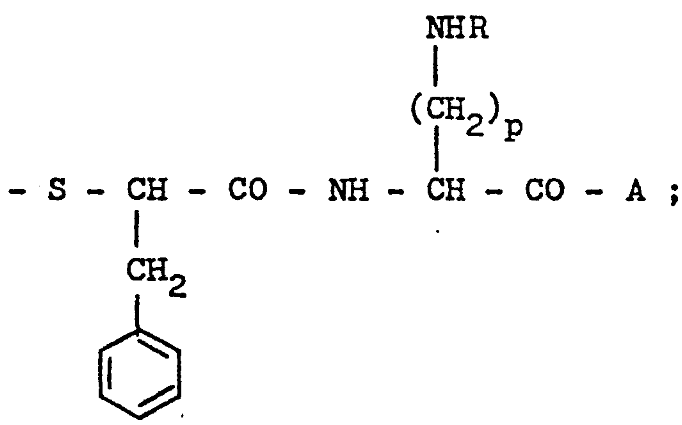

p eine der Zahlen 1 - 4;

A den Rest einer der α-Aminosäuren

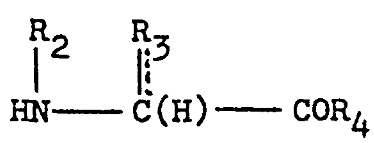

oder

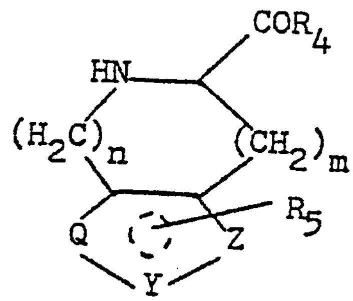

$R_2$ Wasserstoff, $C_1$-$C_4$-Alkyl, gerade oder verzweigt, $C_5$-$C_7$-Cycloalkyl, wobei ein Phenylring ankondensiert sein kann, Phenyl, Phenyl-$C_1$-$C_4$-alkyl, einen Hetero-$C_1$-$C_4$-alkylrest, wobei der Heterocyclus aus einem 5- oder 6-Ring mit 1 - 2 der Heteroatome O, S oder N besteht,

$R_3$ Wasserstoff, $C_1$-$C_4$-Alkyl, gerade oder verzweigt, Phenyl, Phenyl-$C_1$-$C_4$-alkyl oder einen Hetero-$C_1$-$C_4$-alkylrest, wobei der Heterocyclus aus einem 5- oder 6-Ring mit 1 - 2 der Heteroatome O, S oder N besteht, oder

$R_2$ und $R_3$ zusammen bilden mit dem N- und dem C-Atom einen 5-, 6- oder 7-gliedrigen Ring, der gesättigt sein oder eine Doppelbindung enthalten kann, oder einen 4-, 5- oder 6-gliedrigen Ring, der ein oder zwei weitere der Heteroatome O, S oder N enthält;

$R_4$ OH, $C_1$-$C_4$-ω-Hydroxyalkyl, $C_1$-$C_4$-Alkoxy, Phenyl-$C_1$-$C_4$-alkoxy, $C_1$-$C_4$-Alkylamino, $(C_1$-$C_4)_2$-Dialkylamino, eine der Gruppen

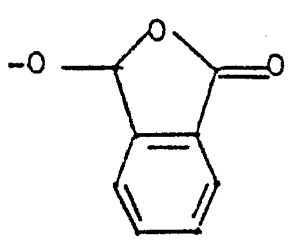

,

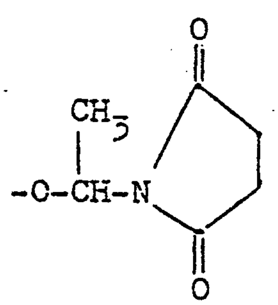

oder

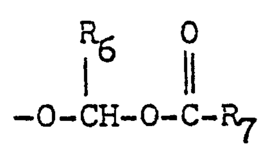

oder

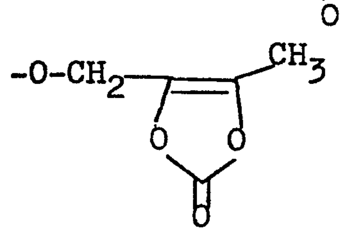

oder eine α-Aminosäure, die peptidartig mit der CO-Gruppe des Moleküls verknüpft ist;

$R_5$ $C_1$-$C_4$-Alkyl, $C_1$-$C_3$-Alkenyl, $C_1$-$C_3$-Alkinyl, gerade oder verzweigt, Hydroxy, Nitro, Amino, $C_1$-$C_4$-Alkoxy, Mercapto, $C_1$-$C_4$-Alkylthio, Hydroxy-$C_1$-$C_4$-alkyl, Mercapto-$C_1$-$C_4$-alkyl, F, Cl, Br, Amino-$C_1$-$C_4$-alkyl, Sulfonamido, Methylendioxy, Fluor-$C_1$-$C_4$-alkyl, Chlor-$C_1$-$C_4$-alkyl, Brom-$C_1$-$C_4$-alkyl, Cyano oder Trifluormethyl;

$R_6$ Wasserstoff oder Methyl;

$R_7$ $C_1$-$C_4$-Alkyl, gerade oder verzweigt, wobei der Alkylrest durch F, Cl, Br, $CF_3$, Phenyl oder Pyridyl substituiert sein kann;

Q, Y und Z O, S, $NR_8$, $CR_9$, $CHR_9$,

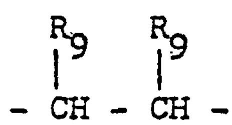 oder 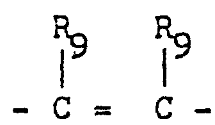 ,

mit der Maßgabe, daß nur einer der Reste X, Y und Z O, S

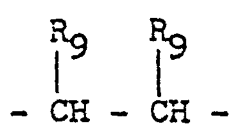 oder 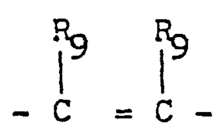 und

einer oder zwei der Reste X, Y und Z $NR_8$ bedeuten können;

$R_8$ Wasserstoff oder $C_1$-$C_4$-Alkyl, gerade oder verzweigt;

$R_9$ Wasserstoff, oder zusammen mit einem vicinal stehenden Rest $R_9$ einen Phenylring oder, für m und n = 1, dessen Dihydroform mit der Doppelbindung in Konjugation zur C-terminalen Carboxygruppe und

m und n jeweils 0,1 oder 2, wobei die Summe aus m und n 1 oder 2 ist.

Bedeuten die Reste $R_2$ und/oder $R_3$ einen 5- oder 6-gliedrigen Hetero-Alkylrest, so seien als Beispiele für den Heterocyclus genannt Thiophen, Furan, Pyrrol, Thiazol, Oxazol, Isoxazol, Pyrazol, Imidazol, Pyran, Thiopyran, Pyridin, Morpholin, Pyrazin, Pyrimidin, Pyridazin, Oxathiin sowie deren Di- und Tetrahydroformen.

Die als Bedeutung für den Rest $R_4$ genannten, gegebenenfalls substituierten Alkylgruppen können gerade oder verzweigt sein; als α-Aminosäuren, die peptidartig mit der CO-Gruppe des Moleküls verbunden sein können, kommen insbesondere in Betracht Prolin, Valin, Leucin, Isoleucin und Phenylalanin.

Bei einer der Bedeutungen für A können die an die Pyrrolidin- beziehungsweise Piperidincarbonsäure ankondensierten 5- oder 6-Ring-Heterocyclen gesättigt oder ungesättigt sein. Bevorzugte Heterocyclen sind Furan, Pyrrol, Thiophen, Benzofuran, Indol, Benzothiophen, Oxazol, Imidazol, Thiazol, Isoxazol, Pyrazol, Pyrrolidin, Tetrahydrofuran, Tetrahydrothiophen, Pyridin, Pyridazin, Chinolin, Isochinolin oder Piperidin.

Die neuen Verbindungen weisen im allgemeinen mehrere Asymmetriezentren auf und liegen daher als Diastereomere oder in Form ihrer Racemate beziehungsweise ihrer racemischen Gemische vor. Die Erfindung umfaßt sowohl die racemischen Gemische als auch die einzelnen Diastereomeren. Bevorzugt sind diejenigen Enantiomeren, bei denen die asymmetrischen C-Atome in der S-Konfiguration vorliegen.

Die erwähnten Racemate können in üblicher Weise, z.B. durch fraktionierte Kristallisation, durch chemische oder durch biochemische Spaltung, in ihre sterisch einheitlichen Formen angereichert oder rein erhalten werden.

Die erfindungsgemäßen Verbindungen liegen in Form ihrer Betaine vor. Sie sind daher befähigt, Salze sowohl mit Säuren als auch mit Basen zu bilden. Beispiele für anorganische Basen sind Ammoniak, Alkalimetallhydroxyde wie Natrium- oder Kaliumhydroxyd oder Erdalkalimetallhydroxyde wie Calcium- oder Magnesiumhydroxyd; an organischen Basen seien genannt N,N'-Dibenzyläthylendiamin oder Triäthylamin. Als Beispiele für zur Salzbildung geeignete Säuren seien genannt Halogenwasserstoffsäuren, Schwefelsäure oder Sulfonsäuren.

Die neuen Stoffe der allgemeinen Formel I können nach verschiedenen Verfahren erhalten werden:

a) Durch Kondensation einer Säure der allgemeinen Formel

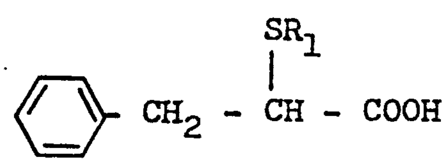

(II)

worin

$R_1$ die oben angegebene Bedeutung besitzt,

mit einem Dipeptid der allgemeinen Formel

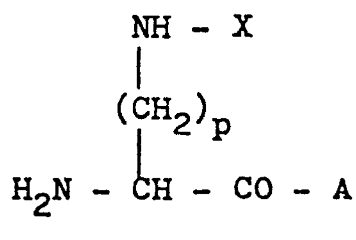

(III)

worin
A und p die oben angegebene Bedeutung besitzen und
X eine geeignete Schutzgruppe, z.B. die t-Butyloxycarbonyl-, α,α-Dimethyl-3,5-dimethoxy-oxycarbonyl(Ddz)-, Adamantyloxycarbonyl(Adoc)-, 2-(p-Biphenylyl)-isopropyloxycarbonyl(Bpoc)-, t-Amyloxycarbonyl(Aoc)-, Furfuryloxycarbonyl(Foc)- oder p-Methoxybenzyloxycarbonyl(Z(omet)-gruppe bedeutet.

b) Kondensation einer Verbindung der allgemeinen Formel

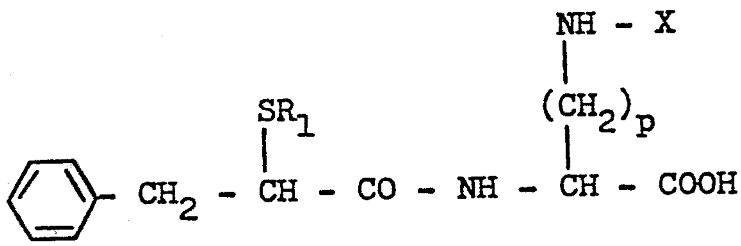
(IIa)

worin
$R_1$, X und p die oben angegebene Bedeutung haben,
mit einer Aminosäure A, wobei A die oben angegebene Bedeutung besitzt.

Die Verbindung der Formel IIa kann gegebenenfalls durch peptidische Verknüpfung einer Verbindung der allgemeinen Formel II mit einer Verbindung der allgemeinen Formel

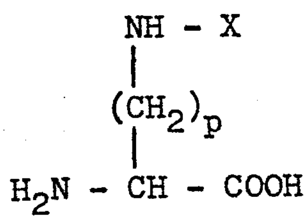
(IV)

erhalten werden.

c) Umsetzung einer Verbindung der allgemeinen Formel

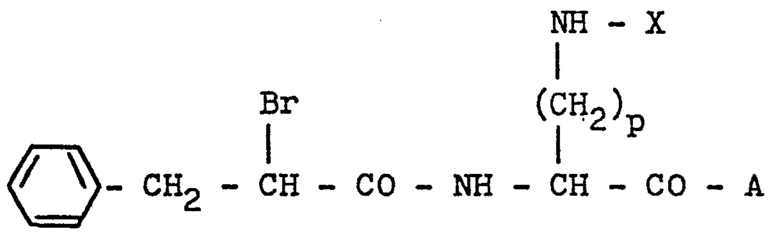

(V)

worin
X, p und A die oben angegebene Bedeutung besitzen, mit einer Mercaptoverbindung der allgemeinen Formel

$$HS - R_1 \qquad (VI)$$

worin $R_1$ die oben angeführte Bedeutung besitzt.

Die Verbindung der Formel (V) kann durch Kondensation von 3-Phenyl-2-brompropionsäure mit einem Dipeptid der allgemeinen Formel III erhalten werden.

d) Kondensation einer Verbindung der allgemeinen Formel

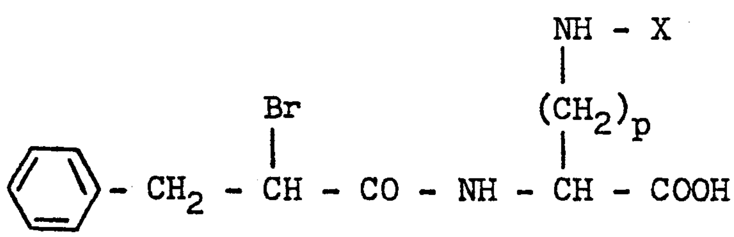

(IVa)

worin
X und p die oben angeführte Bedeutung besitzen, mit einer Aminosäure A, wobei A die vorstehend angegebene Bedeutung besitzt und anschließender Umsetzung des so erhaltenen Kondensationsproduktes der allgemeinen Formel V mit einer Mercaptoverbindung der allgemeinen Formel VI. Die Verbindung der Formel IVa kann durch peptidartige Verknüpfung von 3-Phenyl-2-brompropionsäure mit einer Verbindung der allgemeinen Formel IV erhalten werden.

e) eine Verbindung gemäß Formel I in welcher $R_1$ Acetyl, Benzoyl oder Pivaloyl bedeutet zur entsprechenden Verbindung gemäß Formel I in welcher $R_1$ Wasserstoff ist, umgewandelt wird

f) eine Verbindung gemäß Formel I in welcher $R_1$ Acetyl, Benzoyl oder Pivaloyl bedeutet und R Wasserstoff bedeutet, zur entsprechenden Verbindung gemäß Formel I in welcher $R_1$ Wasserstoff ist und R Acetyl, Benzoyl oder Pivaloyl ist, umgewandelt wird,und daß man etwa vorhandene Schutzgruppen einer geeigneten Stufe der Verfahren a), b), c), d), e) oder f) abgespalten werden.

Die vorstehend beschriebenen Kondensationen erfolgen nach allgemein üblichen Methoden, wie sie in Houben-Weyl, Band XV/1, 4. Auflage, 1974 für die Synthese von Peptiden beschrieben worden sind.
Im allgemeinen benutzt man die in der Peptidchemie üblichen Kupplungsreaktionen unter Verwendung einer aktivierten Form der Säuren II, IIa, IV und IVa; als carboxylaktivierende Gruppen kommen in Frage Säurechloride, Azide, Anhydride, p-Nitrophenylester, Trichlorphenylester, Thiophenylester, o-Cyanomethylester usw.. Als Kupplungsmittel werden bevorzugt Carbonyldiimidazol, Dicyclohexylcarbodiimid, Äthoxyacetylen, Diphenylphosphorylazid oder Chlorameisensäureester; die Umsetzung erfolgt bei Temperaturen zwischen $0^oC$ und + $10^oC$.

Im vorliegenden Fall hat sich die Verwendung von Methylenchlorid als Lösungsmittel zusammen mit Dicyclohexylcarbodiimid und Triäthylamin als vorteilhaft erwiesen.

Die Umsetzung der Brom-Zwischenprodukte mit einer Mercaptoverbindung der allgemeinen Formel VI erfolgt vorteilhaft in Äther als Lösungsmittel. Durch die Umsetzung mit Thiolsäuren (z.B. Thiolessigsäure) werden die entsprechenden Acylmercaptoverbindungen erhalten; die Acylgruppe kann mit starken Basen abgespalten werden.

Die Umwandlung gemäß Verfahren f) erfolgt vorteilhaft nach üblichen Methoden, z.B. durch Umsetzung der Verbindung gemäß Formel I mit starken Basen und Neutralisierung des resultierenden Produktes. Dieses Verfahren kann in wässriger Lösung bei Raumtemperatur durchgeführt werden.

Dimere der allgemeinen Formel I

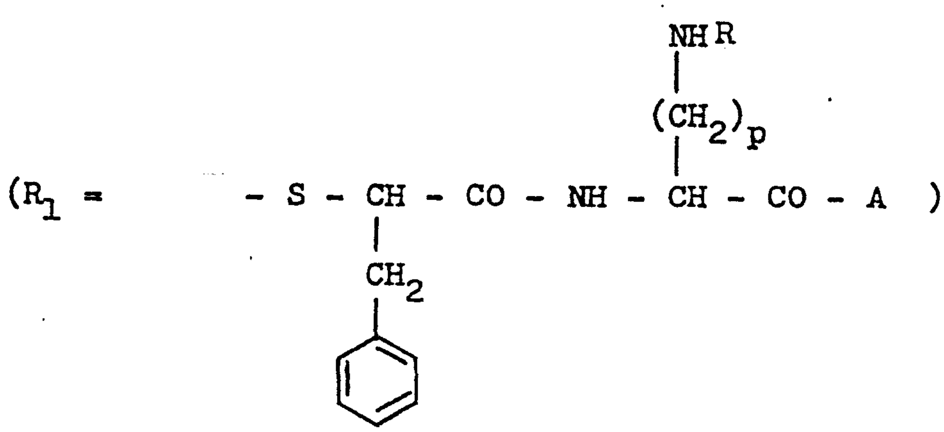

erhält man beispielsweise

α) durch Oxydation einer Verbindung der allgemeinen Formel I, in der $R_1$ Wasserstoff bedeutet und die $NH_2$-Gruppe durch eine der vorstehend für X aufgeführten Gruppen geschützt ist, mit einem Oxydationsmittel wie Sauerstoff oder Jod;

β) durch Umsetzung einer Verbindung der allgemeinen Formel IVa mit Thiolessigsäure zu einer Verbindung IIa und anschließender Verseifung zu einer Verbindung mit

freier Mercaptogruppe. Die Oxydation zum entsprechenden Dimeren erfolgt wie unter α) beschrieben; das erhaltene Zwischenprodukt wird, wie unter Verfahren b) beschrieben, mit einer Aminosäure A kondensiert.

Bevorzugt sind solche Verbindungen der allgemeinen Formel I, in denen $R_1$ Wasserstoff, p = 4 und A Prolin oder Thiazolidin bedeutet.

Nach den oben beschriebenen Verfahren können die folgenden Endprodukte, gegebenenfalls in Form ihrer Salze, erhalten werden:

N-[$N^{\alpha}$-(3-Phenyl-2-S-acetylmercaptopropanoyl)-S-lysyl]-S-prolin

N-[$N^{\alpha}$-(3-Phenyl-2-S-mercaptopropanoyl)-S-lysyl]-S-prolin

N-[$N^{\alpha}$-(3-Phenyl-2-R-mercaptopropanoyl)-S-lysyl]-S-prolin

N-[$N^{\alpha}$-(3-Phenyl-2-S-mercaptopropanoyl)-S-lysyl]-thiazolidin-4-S-carbonsäure

N-[$N^{\alpha}$-(3-Phenyl-2-S-acetylmercaptopropanoyl)-S-lysyl]-thiazolidin-4-S-carbonsäure

N-[$N^{\alpha}$-(3-Phenyl-2-S-acetylmercapto)-S-lysyl]-S-prolin-pivaloyloxymethylester

N-[$N^{\alpha}$-(3-Phenyl-2-S-acetylmercapto)-S-lysyl]-thiazolidin-4-S-carbonsäure-pivaloyloxymethylester

N,N'-[N,N'-{2,2'-S-Dithiobis-(3-phenylpropanoyl)}-bis-S-lysyl]-bis-S-prolin

N,N'-[N,N'-{2,2'-S-Dithiobis-(3-phenylpropanoyl)}-bis-S-lysyl]-bis-S-prolinpivaloyloxymethylester

N,N-[N,N'-{2,2'-S-Dithiobis-(3-phenylpropanoyl)}-bis-S-lysyl]-bis-thiazolidin-4-S-carbonsäure

N,N-[N,N'-{2,2'-S-Dithiobis-(3-phenylpropanoyl)}-bis-S-lysyl]-bis-thiazolidin-4-S-carbonsäure-pivaloyloxymethylester

Bei den Ausgangsstoffen handelt es sich meist um bekannte Verbindungen, auch eventuell neue Ausgangsstoffe werden nach an sich bekannten Verfahren hergestellt; Verbindungen der allgemeinen Formel III erhält man z.B. durch peptidartige Verknüpfung einer Verbindung der Formel

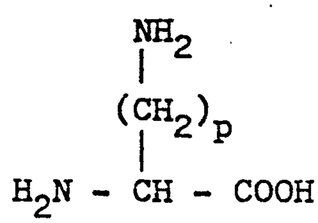

mit einer Aminosäure A.

Die Aminosäurederivate der allgemeinen Formel I besitzen eine starke, lang anhaltende blutdrucksenkende Wirkung. Diese beruht auf einer Hemmung des Angiotensin I Converting Enzyms und damit einer Blockierung der Bildung des Vasokonstriktors Angiotensin II aus Angiotensin I. Darüber hinaus wirken die neuen Verbindungen hemmend auf das für den Bradykininabbau verantwortliche Enzym Kininase II, das als identisch mit dem oben genannten Converting Enzym gilt. Da Bradykinin eine gefäßerweiternde Wirkung besitzt, wird der blutdrucksenkende

Effekt durch diese zusätzliche Wirkung verstärkt. Die durch Bradykinin erzeugte Blutdrucksenkung an normalen Ratten wird durch die neuen Verbindungen verstärkt. Ausdruck dieser Wirkung könnte auch die an unvorbehandelten genetischen Hochdruckratten beobachtete blutdrucksenkende Wirkung sein.

So erzeugt N-[$N^{\alpha}$-(3-Phenyl-2-S-mercaptopropanoyl)-S-lysyl]-thiazolidin-4-S-carbonsäure an der narkotisierten Ratte in einer Dosierung von 0,3 mg/kg i.v. eine lang anhaltende Blutdrucksenkung von 60 %, wenn man vorher durch eine i.v.-Gabe von 0,1 mg/kg Angiotensin I den Blutdruck steigert; beim N-[$N^{\alpha}$-(3-Phenyl-2-S-mercaptopropanoyl)S-lysyl]-S-prolin liegt der Wert bei gleichem Test bei 57 %.

N-[$N^{\alpha}$-(3-Phenyl-2-S-mercaptopropanoyl)-S-lysyl]-thiazolidin-4-S-carbonsäure zeigt am Angiotensin I Converting Enzym in vitro eine Hemmaktivität $IC_{50} = 4{,}6 \cdot 10^{-9}$ [M]; die $IC_{50}$ beträgt beim N-[$N^{\alpha}$-(3-Phenyl-2-S-mercaptopropanoyl)-S-lysyl]-S-prolin $2{,}7 \cdot 10^{-8}$ [M].

Als $IC_{50}$ wird hierbei diejenige Hemmstoffkonzentration bezeichnet, bei der das Angiotensin I Converting Enzym zu 50 % gehemmt wird (s. H. S. Cheung, D. W. Cushman, Biochem. Biophys. Acta 293, 451 (1973)).

Für die Anwendung in der Therapie werden die neuen Verbindungen mit üblichen pharmazeutischen Füll- oder Trägerstoffen, Streck-, Spreng-, Binde-, Gleit-, Dickungs- oder Verdünnungsmitteln gemischt.
Als pharmazeutische Zubereitungsformen kommen zum Beispiel Tabletten, Kapseln, Zäpfchen, Lösungen, Säfte, Emulsionen oder dispersible Pulver in Frage, wobei gewünschtenfalls weitere bekannte Wirkstoffe, z.B. Saluretica, Diuretica und/oder Antihypertonica zugefügt werden können.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Äthylendiamintetraessigsäure und unter Zugabe geeigneter Lösungsvermittler hergestellt und in Injektionsflaschen oder Ampullen abgefüllt.

Die einen oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Die tägliche Dosis für die Anwendung der erfindungsgemäßen Verbindungen der allgemeinen Formel I beziehungsweise ihre Salze soll 5 - 500 vorzugsweise 10 - 100 mg betragen und kann in 1 - 4 Einzeldosen verabreicht werden.

Die folgenden Beispiele dienen zur näheren Erläuterung der Erfindung. Die Struktur aller synthetisierter Beispiele wurde durch NMR-Spektren bestätigt.

Beispiel 1

N-[$N^{\alpha}$-(3-Phenyl-2-S-acetylmercaptopropanoyl)-S-lysyl]-S-prolin

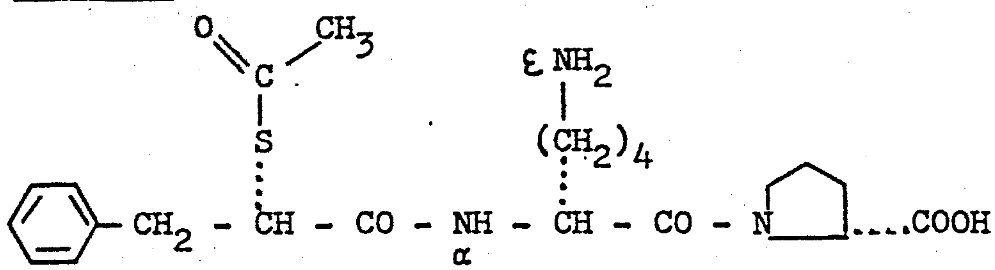

10,7 g (26,7 mMol) N-($N^{\varepsilon}$-tert.Butoxycarbonyl-S-lysyl)-S-prolin-tert.butylester und 6,11 g (26,7 mMol) 3-Phenyl-2-R-brompropionsäure werden in 270 ml wasserfreiem Dichlormethan gelöst und bei 0°C unter Rühren 5,5 g (26,7 mMol) N,N'-Dicyclohexylcarbodiimid zugegeben. Man rührt über Nacht bei Raumtemperatur weiter, saugt vom ausgefallenen Harnstoffderivat ab und engt das Filtrat im Vakuum ein. Der Rückstand wird in Essigester gelöst und vom Ungelösten abfiltriert. Die organische Phase wird nacheinander mit 5%iger $KHSO_4$-Lösung, $NaHCO_3$-Lösung und gesättigter NaCl-Lösung gewaschen und über $MgSO_4$ getrocknet. Nach Abdestillieren des Essigesters i.V. bleibt ein öliger Rückstand von 14 g (= 85,8 % d.Th.), dem NMR-spektroskopisch die Struktur des N-[$N^{\alpha}$-(3-Phenyl-2-R-brompropanoyl)-$N^{\varepsilon}$-tert. butoxycarbonyl-S-lysyl]-S-prolin-tert.butylesters zukommt.
Für den Acetylmercapto-Austausch werden 3,48 g (45,8 mMol) Thiolessigsäure in 120 ml wasserfreiem Ether gelöst und 4,62 g (45,8 mMol) Triethylamin, dann 14 g (22,9 mMol) der aus dem obigen Ansatz erhaltenen Bromverbindung bei 0°C zugetropft. Man kocht noch 90 Minuten am Rückfluß und läßt über Nacht bei Raumtemperatur stehen. Vom ausgefallenen Triethylaminhydrobromid wird abgesaugt, das Filtrat i.V. abdestilliert und dabei 15,5 g eines braunen Öls als Rückstand erhalten.

Die chromatographische Reinigung über Kieselgel (Laufmittel: Essigester, n-Hexan = 1:1) ergab 9,4 g (= 67,7 % d.Th.) des N-[$N^{\alpha}$-(3-Phenyl-2-S-acetyl-mercaptopropanoyl)-$N^{\epsilon}$-tert.butoxycarbonyl-S-lysyl]-S-prolin-tert.butylesters.

Zur Verseifung des tert.Butylesters und gleichzeitiger Abspaltung der $N^{\epsilon}$-tert.Butoxycarbonyl-Schutzgruppe werden 5,5 g (9,08 mMol) des aus vorstehendem Ansatz gewonnenen Esters in 150 ml Trifluoressigsäure gelöst und bei Raumtemperatur über Nacht gerührt. Nach Abdestillieren der Trifluoressigsäure i.V. wird noch mehrmals in Dichlormethan gelöst und eingedampft. Der Rückstand wird in $NaHCO_3$-Lösung gelöst und mit Essigester ausgeschüttelt; die wäßrige Phase wird mit konzentrierter Salzsäure angesäuert und zweimal mit Dichlormethan extrahiert. Die vereinigten Dichlormethanextrakte werden mit Wasser und gesättigter NaCl-Lösung gewaschen, getrocknet und im Vakuum abdestilliert. Rückstand 0,5 g. Die wäßrige Phase wird mit NaCl gesättigt und erneut mit Dichlormethan extrahiert. Nach Trocknen über $MgSO_4$ und Eindampfen werden weitere 3 g, zusammen also 3,5 g, als erstarrter Schaum erhalten, der NMR-spektroskopisch der Struktur der Titelverbindung entspricht.
Rf = 0,22 (Butanol, Essigsäure, Wasser = 3:1:1, Silicalgel).

Beispiel 2

N-[$N^{\alpha}$-(3-Phenyl-2-S-mercaptopropanoyl)-N -acetyl-S-lysyl]-S-prolin

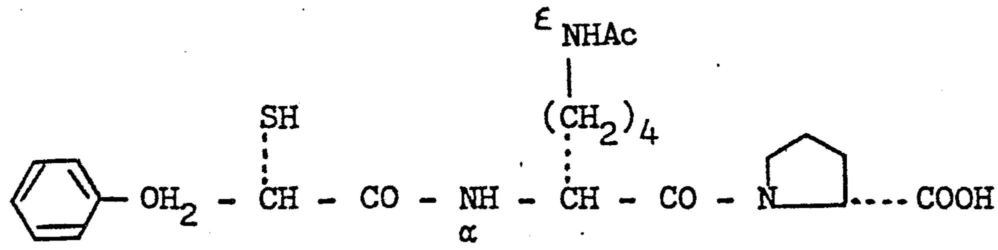

4,2 g (9,3 mMol) N-[$N^{\alpha}$-(3-Phenyl-2-S-acetylmercapto-propanoyl)-S-lysyl]-S-prolin werden in 95 ml Wasser gelöst, mit 8,2 ml 5 n Natronlauge versetzt und 1,5 Stunden bei Raumtemperatur und $N_2$-Atmosphäre gerührt. Danach werden weitere 4,1 ml 5 n Natronlauge zugegeben und 1,5 Stunden weitergerührt. Die alkalische Lösung wird mit Essigester ausgeschüttelt, mit konzentrierter Salzsäure angesäuert und anschließend mit Dichlormethan extrahiert. Die organische Phase wird mit gesättigter NaCl-Lösung gewaschen, getrocknet und i.V. abdestilliert. Es werden 2,1 g (= 55,2 % d.Th.) der Titelverbindung als weißer erstarrter Schaum erhalten.
Rf = 0,49 (Butanol, Essigsäure, Wasser = 3:1:1,
(Kieselgel $60F_{254}$Merck).

Beispiel 3

N-[$N^{\alpha}$-(3-Phenyl-2-S-mercaptopropanoyl)-S-lysyl]-thiazolidin-4-S-carbonsäure

3,5 g (7,6 mMol) $N^{\alpha}$-(3-Phenyl-2-R-brompropanoyl)-$N^{\varepsilon}$-tert.butoxycarbonyl-S-lysin und 1,4 g (7,6 mMol) Thiazolidin-4-S-carbonsäuremethylester-hydrochlorid werden in 70 ml wasserfreiem Dichlormethan gelöst und unter Rühren und Kühlen auf 0°C 769 mg (7,6 mMol) Triethylamin, dann 1,6 g (7,6 mMol) N,N'-Dicyclohexylcarbodiimid zugegeben. Man rührt bei Raumtemperatur ca. 17 Stunden lang, saugt vom ausgeschiedenen Dicyclohexylharnstoff ab und destilliert das Lösungsmittelab. Der ölige Rückstand wird in Essigester gelöst, nach Kühlung erneut abfiltriert und die organische Phase nacheinander mit verdünnter $KHSO_4$-Lösung, gesättigter $NaHCO_3$-Lösung, Wasser und gesättigter NaCl-Lösung gewaschen. Nach Trocknen über $MgSO_4$ wird das Lösungsmittel i.V. abdestilliert. 4,1 g des rohen, öligen Reaktionsproduktes werden über Kieselgel mit Essigester/n-Hexan = 1:1 als Elutionsmittel chromatographisch gereinigt und dabei 2,9 g (= 68,8 % d.Th.) N-[$N^{\alpha}$-(3-Phenyl-2-R-brompropanoyl)-$N^{\varepsilon}$-tert.butoxycarbonyl-S-lysyl]-thiazolidin-4-S-carbonsäuremethylester in Form eines erstarrten Schaumes erhalten, der aus Ether kristallisierte.
Rf = 0,43 (Essigester, n-Hexan = 2:1, Silicagel).

Zum Austausch des Broms gegen die Acetylmercaptogruppe wird eine Lösung von 792 mg (10,4 mMol) Thiolessigsäure in 35 ml wasserfreiem Ether unter Stickstoff und Eiskühlung langsam mit 1,05 g (10,4 mMol) Triethylamin versetzt und anschließend tropfenweise unter Rühren zu einer Lösung von 2,9 g (5,2 mMol) der oben genannten Bromverbindung in 20 ml Essigester gegeben.

Man kocht danach noch 60 Minuten am Rückfluß, filtriert die vorher gekühlte Lösung und wäscht nacheinander mit verdünnter $KHSO_4$-Lösung, $NaHCO_3$-Lösung, Wasser und gesättigter NaCl-Lösung. Nach dem Trocknen über $MgSO_4$ wird das Lösungsmittel im Vakuum abdestilliert und als Rückstand 3,3 g eines gelblichen Öles erhalten. Die chromatographische Reinigung erfolgt über 100 g Kieselgel mit Essigester/n-Hexan = 2:1 als Elutionsmittel; es werden so 1,9 g (64,1 % d.Th.) reiner N-[$N^{\alpha}$-(3-Phenyl-2-S-acetylmercaptopropanoyl)-$N^{\varepsilon}$-tert.-butoxycarbonyl-S-lysyl]-thiazolidin-4-S-carbonsäuremethylester als erstarrter Schaum erhalten.
Rf = 0,36 (Essigester, n-Hexan = 2:1, Silicagel).

Zur Abspaltung der tert.Butoxycarbonyl-Schutzgruppe werden 1,9 g (3,33 mMol) der vorstehenden Acetylmercaptoverbindung mit 40 ml Trifluoressigsäure 3 Stunden bei Raumtemperatur gerührt, anschließend im Vakuum zur Trockne eingedampft und zur Entfernung restlicher Trifluoressigsäure nacheinander je dreimal in Aceton und in Chloroform gelöst und erneut eingedampft. Es werden 2,7 g N-[$N^{\alpha}$-(3-Phenyl-2-S-acetylmercaptopropanoyl)-S-lysyl]-thiazolidin-4-S-carbonsäuremethylester in Form eines zähen Öles erhalten.
Rf = 0,23 (Chloroform, Methanol, Eisessig = 90:10:5, Silicagel).

Zur gleichzeitigen Verseifung des Methyl- und des Thiolessigsäureesters werden die 2,7 g (3,3 mMol) des vorstehenden Reaktionsproduktes in 20 ml Methanol und 40 ml Wasser gelöst und mit 4 ml 5 n Natronlauge versetzt. Nach 1,5-stündigem Rühren werden 2 ml 5 n Natronlauge bei Raumtemperatur zugesetzt und nochmals 1,5 Stunden weitergerührt. Das Methanol wir i.V. abdestilliert, der Rückstand mit Wasser versetzt und mit Dichlormethan ausgeschüttelt.

Die wäßrige Phase wird mit verdünnter Salzsäure angesäuert, das sich ausscheidende zähe Öl im Scheidetrichter abgetrennt und in wenig Wasser gelöst, über 100 g Dowex 50 W x 4 gereinigt (Elution mit 5%igem wäßrigem Pyridin).
700 mg (= 45,9 % d.Th.) N-[$N^{\alpha}$-(3-Phenyl-2-S-mercaptopropanoyl)-S-lysyl]-thiazolidin-4-S-carbonsäure werden NMR-spektroskopisch rein erhalten.
Rf = 0,52 (Essigester, Butanol, Wasser, Essigsäure = 1:1:1:1, Silicagel).

<u>Herstellung der Ausgangsverbindungen:</u>

<u>$N^{\alpha}$-(3-Phenyl-2-R-brompropanoyl)-$N^{\varepsilon}$-tert.butoxycarbonyl-S-lysin</u>

3,25 g (14,2 mMol) 3-Phenyl-2-R-brompropionsäure und 3,7 g (14,2 mMol) $N^{\varepsilon}$-tert.Butoxycarbonyl-S-lysinmethylester werden in 70 ml wasserfreiem Dichlormethan gelöst, unter Rühren und Kühlen (0°C) 2,9 g (14,2 mMol) N,N'-Dicyclohexylcarbodiimid zugegeben und über Nacht bei Raumtemperatur weitergerührt. Man saugt vom ausgeschiedenen Dicyclohexylharnstoff ab, entfernt das Lösungsmittel durch Destillation und nimmt den Rückstand in Essigester auf. Nach Kühlen und erneuter Filtration wird die organische Phase nacheinander mit verdünnter $KHSO_4$-Lösung, gesättigter $NaHCO_3$-Lösung, Wasser und gesättigter NaCl-Lösung gewaschen, über $MgSO_4$ getrocknet und im Vakuum zur Trockne eingedampft. Der kristalline Rückstand (6,2 g) wird aus Essigester und n-Hexan umkristallisiert und dabei 4,8 g (= 71,1 % d.Th.) des Methylesters der Titelverbindung erhalten; Fp. 97 - 98°C.
Rf = 0,49 (Essigester, n-Hexan = 2:1, Kieselgel),
Rf = 0,7 (Chloroform, Methanol, Eisessig = 90:10:5, Silicagel).

Zur Verseifung des voranstehenden Methylesters werden 4,8 g (10,2 mMol) des $N^{\alpha}$-(3-Phenyl-2-R-brompropanoyl)-$N^{\varepsilon}$-butoxycarbonyl-S-lysin-methylesters in 50 ml Methanol gelöst und unter Rühren und Eiswasserkühlung 11 ml 1 n Natronlauge (11 mMol) zugegeben. Nach dreistündigem Rühren bei Raumtemperatur wird das Methanol im Vakuum abgezogen, der wäßrige Rückstand mit Wasser verdünnt und die trübe Lösung mit 5%iger $KHSO_4$-Lösung angesäuert und mit Dichlormethan extrahiert. Das aus der Dichlormethanlösung ausgeschiedene, kristalline Produkt wird abgesaugt (2,32 g) und aus dem Filtrat noch weitere 2 g kristalline Masse erhalten. Beide Fraktionen werden vereinigt und aus Chloroform und wenig Methanol umkristallisiert. 3,1 g (= 66,4 % d.Th.) der Titelverbindung werden erhalten.
Fp. 178 - 179°C (Zers.)
Rf = 0,55 (Chloroform, Methanol, Eisessig = 90:10:5, Silicagel).

## Beispiel 4

### N-[$N^{\alpha}$-(3-Phenyl-2-S-acetylmercaptopropanoyl)-S-lysyl]-thiazolidin-4-S-carbonsäure

Das S-Acylderivat der Titelverbindung aus Beispiel 3 wird erhalten, wenn man, wie im Beispiel 3 beschrieben, anstelle des Thiazolidinmethylesters den entsprechenden Thiazolidin-4-S-carbonsäure-tert.butylester einsetzt und mit Trifluoressigsäure in einem Reaktionsschritt den tert.Butylester des Reaktionsproduktes verseift und gleichzeitig seine N -Schutzgruppe abspaltet.

Beispiel 5

N-[$N^{\alpha}$-(3-Phenyl-2-S-acetylmercapto)-S-lysyl]-S-prolin-pivaloyloxymethylester

3 g (6,6 mMol) N-[$N^{\alpha}$-(3-Phenyl-2-S-acetylmercapto)-S-lysyl]-S-prolin werden in Aceton gelöst, 660 mg (6,6 mMol) $KHCO_3$, 185 mg Kaliumjodid und 1,16 ml (8,06 mMol)Chlormethylpivalat zugegeben und 3 Stunden am Rückfluß erhitzt. Nach Abkühlung wird vom ausgefallenen KCl abfiltriert und das Filtrat eingeengt. Der Rückstand wird in Essigester aufgenommen, nacheinander mit Wasser, verdünnter $NaHCO_3$-Lösung, Wasser und gesättigter NaCl-Lösung gewaschen und über $MgSO_4$ getrocknet. Nach Abdestillieren des Essigesters werden 2,1 g eines öligen Rückstandes erhalten, der über Kieselgel chromatographisch gereinigt wird (Elution: eingangs Essigester, n-Hexan = 2:1, dann Wechsel auf Essigester, Methanol = 9:1).
Es werden 0,8 g eines öligen Produktes erhalten, das NMR-spektroskopisch der Titelverbindung entspricht.
Rf = 0,36 (Essigester, Methanol = 9:1, Silicagel).

Beispiel 6

N-[$N^{\alpha}$-(3-Phenyl-2-S-acetylmercapto)-S-lysyl]-thiazolidin-4-S-carbonsäure-pivaloyloxymethylester

Der der Titelverbindung im Beispiel 5 analoge Thiazolidin-4-S-carbonsäure-pivaloyloxymethylester wird erhalten, wenn man anstelle der Prolinverbindung im Beispiel 5 die N-[$N^{\alpha}$-(3-Phenyl-2-S-acetylmercapto)-S-lysyl]-thiazolidin-4-S-carbonsäure einsetzt.

Beispiel 7

N,N'-[N,N'-{2,2'-S-Dithiobis-(3-phenylpropanoyl)}-bis-S-lysyl]-bis-S-prolin

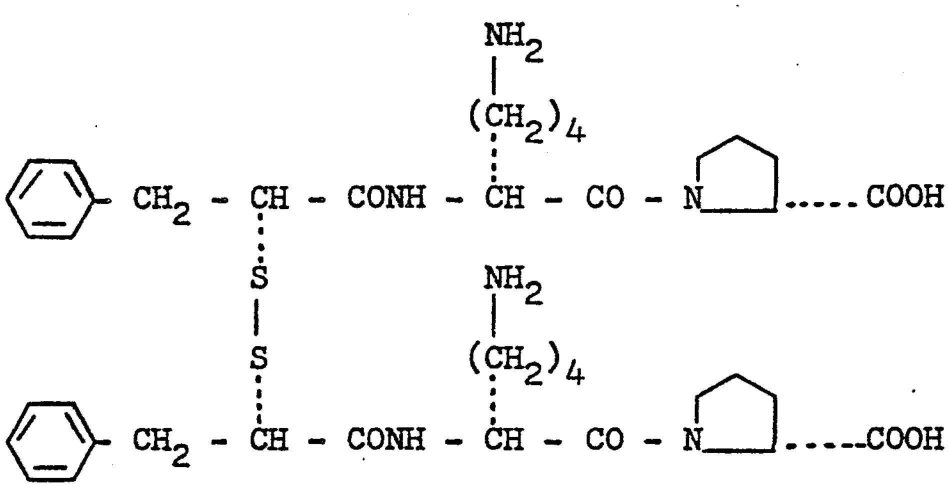

3,9 g (6,43 mMol) N-[$N^{\alpha}$-(3-Phenyl-2-S-acetylmercapto-propanoyl)-$N^{\varepsilon}$-tert.butoxycarbonyl-S-lysyl]-S-prolin-tert.butylester werden in 50 ml Methanol gelöst, mit 10 ml Wasser und 5,6 ml 5 n Natronlauge versetzt und 1,5 Stunden bei Raumtemperatur gerührt. Danach werden 2,8 ml 5 n Natronlauge zugegeben und weitere 1,5 Stunden gerührt. Man destilliert im Vakuum das Methanol ab, löst den Rückstand in 300 ml Dichlormethan und schüttelt mit Wasser aus. Die organische Phase wird nacheinander mit verdünnter $KHSO_4$-Lösung, Wasser und gesättigter NaCl-Lösung gewaschen, über $MgSO_4$ getrocknet und i.V. abdestilliert.
3,4 g (= 94 % d.Th.)der freien Mercaptoverbindung werden als Öl erhalten.

Zur Oxidation werden die 3,4 g (6 mMol) der voranstehenden entacetylierten Verbindung in 100 ml Dichlormethan gelöst, 0,8 g (8 mMol) $KHCO_3$ zugesetzt und eine 2%ige methanolische Jodlösung bei Raumtemperatur bis zur bleibenden Gelbfärbung zugetropft (Verbrauch ca. 25 ml).

Man fügt ca. 50 ml Wasser zu und reduziert das überschüssige Jod mit einigen Tropfen einer $Na_2S_2O_3$-Lösung. Die organische Phase wird abgetrennt, mehrmals mit Wasser und gesättigter NaCl-Lösung gewaschen, über $MgSO_4$ getrocknet und i.V. zur Trockne eingeengt.
2,2 g (=64,9 % d.Th.) der dimeren Bisverbindung werden als erstarrter Schaum erhalten.

Zur Verseifung der Ester- und $N^{\varepsilon}$-Schutzgruppe wird die Bisverbindung (2,2 g = 1,97 mMol) mit 60 ml Trifluoressigsäure übergossen und über Nacht bei Raumtemperatur gerührt. Man destilliert die Trifluoressigsäure i.V. ab, löst in verdünnter $NaHCO_3$-Lösung und schüttelt mit Essigester aus. Danach wird die wäßrige Phase mit verdünnter Salzsäure angesäuert und im Vakuum eingeengt. Der weiße, feste Rückstand wird drei- bis viermal mit Methanol ausgezogen, vom Ungelösten abfiltriert und das Lösungsmittel i.V. zur Trockne eingedampft.
Als Ausbeute werden 1,6 g (= 100 % d.Th.) der Titelverbindung als erstarrter Schaum erhalten.
Rf = 0,2 (Butanol, Essigsäure, Wasser = 3:1:1, Silicagel).

Beispiel 8

N,N'-[N,N'-{2,2'-S-Dithiobis-(3-phenylpropanoyl)}-bis-S-lysyl]-bis-S-prolin-pivaloyloxymethylester

Ausgangsprodukt ist die Titelverbindung aus Beispiel 7. Die Veresterung zur Titelverbindung erfolgt analog der im Beispiel 5 geschilderten Vorgehensweise.

Beispiel 9

N,N-[N,N'-{2,2'-S-Dithiobis-(3-phenylpropanoyl)}-bis-S-lysyl]-bis-thiazolidin-4-S-carbonsäure

Ausgangsverbindung ist der N-[$N^{\alpha}$-(3-Phenyl-2-S-acetylmercaptopropanoyl)-$N^{\varepsilon}$-tert.butoxycarbonyl-S-lysyl]-thiazolidin-4-S-carbonsäure-tert.butylester.
Die Dimerisierung und Schutzgruppenabspaltung zur Titelverbindung erfolgt in der im Beispiel 7 beschriebenen Weise.

Beispiel 10

N,N-[N,N'-{2,2'-S-Dithiobis-(3-phenylpropanoyl)}-bis-S-lysyl]-bis-thiazolidin-4-S-carbonsäure-pivaloyloxymethylester

Ausgangsverbindung ist die Titelverbindung aus Beispiel 9.
Die Veresterung zur Titelverbindung erfolgt analog der im Beispiel 5 geschilderten Weise.

Beispiel 11

N-/$N^{\alpha}$-(3-Phenyl-2-S-mercaptopropanoyl)-S-lysyl/-S-prolin

Zu einer Lösung von 0,989 g (13 mMol) Thiolessigsäure in 45 ml wasserfreiem Ether und 1,31 g (13 mMol) Triethylamin wird tropfenweise unter Stickstoff und Rühren eine Lösung von 3,7 g (6,5 mMol) N-/$N^{\alpha}$-3-phenyl-2-R-brompropanoyl)-$N^{\varepsilon}$-tert.butoxycarbonyl)-S-lysyl/-S-prolin-methylester in 30 ml Essigester gegeben. Man kocht danach am Rückfluß, filtiert die vorher gekühlte Lösung und wäscht nacheinander mit verdünnter $KHSO_4$-Lösung, $NaHCO_3$-Lösung, Wasser und gesättigter NaCl-Lösung. Nach dem Trocknen über $MgSO_4$ wird das Lösungsmittel

im Vakuum abdestilliert und als Rückstand 4,0 g eines gelblichen Öles erhalten. Die chromatographische Reinigung erfolgt über 120 g Kieselgel mit Essigester, n-Hexan = 2:1 als Elutionsmittel; es werden so 3,0 g (81,9 % d. Th.) reiner N-/$N^{\alpha}$-(3-Phenyl-2-S-acetyl-mercaptopropanoyl)-$N^{\varepsilon}$-tert. butoxycarbonylS-lysyl7-S-prolinmethylester als farbloses Öl erhalten. $R_f$ = 0,26 (Essigester, n-Hexan = 2:1, Kieselgel $60_{254}$ Merck).

Durch Schutzgruppenabspaltung wie in Beispiel 3 beschrieben, wird 1,7 g (=78,7 % d. Th.) des Titelprodukts erhalten.

$R_f$ = 0,52 (Essigester, Butanol, Wasser, Essigsäure = 1:1:1:1, Kieselgel $60F_{254}$ Merck).

## Beispiel 12

## N-[$N^{\alpha}$-(3-Phenyl-2-R-mercaptopropanoyl)-S-lysyl]-S-prolin

Das dem Beispiel 11 analoge RSS-Diastereomer wird erhalten, wenn man der R-Bromverbindung das entsprechende S-Isomer einsetzt, nämlich der N-[$N^{\alpha}$-(3-Phenyl-2-S-brompropanoyl)-$N^{\varepsilon}$-tert. butoxycarbonyl-S-lysyl]-S-prolin methylester. $R_f$= 0,44 (n-Butanol, Eisessig, Wasser = 3:1:1 Kieselgel $60F_{254}$ Merck).

## Beispiel 13

## N-[$N^{\alpha}$-(3-Phenyl-2-S-acetylmercaptopropanoyl)-S-lysyl]-S-prolin-(S-methyl-2-oxo-1,3-dioxolen-4-yl)-methylester

A. 2,6 g (4,7 mMol) N-[$N^{\alpha}$-(3-Phenyl-2-R-bromopropanoyl)-$N^{\varepsilon}$-tert. butoxycarbonyl-S-lysyl]-S-prolin werden in 400 ml Aceton gelöst und nacheinander 500,5 mg (5 mMol) $KHCO_3$, 141 mg (0,85 mMol) KJ und 965 mg (5 mMol) 4-Brommethyl-S-methyl-2-oxo-1,3-dioxolen zugegeben. Die Lösung wird in einer $N_2$-atmosphäre unter Rühren 3 Stunden am Rückfluß gekocht und nach abkühlen vom Umgelösten abfiltiert.

Nach Abdestillieren des Lösungsmittels wird der Rückstand in Essigester gelöst und mit Wasser, gesättigter $NaHCO_3$-Lösung, Wasser und gesättigter NaCl-Lösung gewaschen. Nach Trocknen über $MgSO_4$ wird der Essigester im Vakuum abdestilliert. Der Rückstand von 2,8 g wird über 90 g Kieselgel chromatographisch gereinigt (Elution mit Essigester, n-Hexan = 2:1). Es werden 2,3 g (= 73,4 % d. Th.) eines öligen Produktes erhalten, das NMR-spektroskopisch dem Prolin--(5-methyl-2-oxo1,3-dioxolen-4-yl)-methylester derivat entspricht.

$R_f$ = 0,68 (Essigester, n-Hexan = 2:1,
Kieselgel $60F_{254}$ Merck)

B. Zum Austausch des Broms gegen die Acetylmercaptogruppe wird eine Lösung von 525 mg (6,9 mMol) Thiolessigsäure in 20 ml wasserfreiem Ether unter $N_2$-Atmosphäre und Eiskühlung langsam mit 0,7 g (6,9 mMol) Triäthylamin versetzt und anschließend tropfenweise unter Rühren zu einer Lösung von 2,3 g (3,5 mMol) der vorstehend genannten Bromverbindung in 20 ml wasserfreiem Ether und 10 ml Essigester gegeben. Man kocht danach noch 90 Minuten am Rückfluß, filtriert die vorher gekühlte Lösung und wäscht nacheinander mit verdünnter $KHSO_4$-Lösung, $NaHCO_3$-Lösung, Wasser und gesättigter NaCl-Lösung. Nach dem Trocknen über $MgSO_4$ wird das Lösungsmittel im Vakuum abdestilliert und als Rückstand 2,3 g eines gelblichen Öls erhalten. Die chromatographische Reinigung erfolgt über 7,5 g Kieselgel mit Essigester, n-Hexan = 2:1 als Elutionsmittel. Es werden so 1,7 g (= 74,4 % d. Th.) von N-[$N^{\alpha}$-(3-Phenyl-2-S-acetylmercapto-propanoyl)-$N^{\varepsilon}$-tert.butoxycarbonyl-S-lysyl]-S-prolin-(5-methyl-2-oxo-1,3-dioxolen-4-yl)-methylester.

C. Zur Abspaltung der tert. butoxycarbonyl-$N^{\varepsilon}$ Schutzgruppe des Lysins werden 1,7 g (2,57 mMol) des Produkts aus Stufe B mit 40 ml Trifluoressigsäure 3 Stunden bei Raumtemperatur gerührt, anschließend im Vakuum zum Trocknen eingedampft und zur Entfernung restlicher Trifluoressigsäure nacheinander je dreimal in Aceton und in Chloroform gelöst und erneut eingedampft. Es werden 2,3 g der Titelverbindung erhalten.

Beispiel 14

Die Titelverbindung aus den Beispielen 1 und 2 analog RSS-Diastereomeren nämlich N-/$N^{\alpha}$-3-Phenyl-2-R-acetylmercaptopropanoyl)-S-lysyl7-S-prolin und N-/$N^{\alpha}$-3-Phenyl-2-R-mercaptopropanoyl)-$N^{\varepsilon}$-acetyl-S-lysyl7-S-prolin werden erhalten, wenn man anstelle der R-Bromverbindung das entsprechende S-Isomere einsetzt, nämlich die 3-Phenyl-2-S-brompropionsäure.

0176999

## Pharmazeutische Anwendungsbeispiele

### a) Dragees

1 Drageekern enthält:

| | |
|---|---|
| Wirkstoff gemäß Anspruch 1 | 10,0 mg |
| Milchzucker | 60,0 mg |
| Maisstärke | 35,0 mg |
| Gelatine | 3,0 mg |
| Magnesiumstearat | 2,0 mg |
| | 110,0 mg |

Herstellung:

Die Mischung der Wirksubstanz mit Milchzucker und Maisstärke wird mit einer 10%igen wässrigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässrigen Suspension von Zucker, Titandioxyd, Talkum und Gummi arabicum aufgebracht wird. Die fertigen Dragees werden mit Bienenwachs poliert.

b) Tabletten

| | |
|---|---|
| Wirkstoff gemäß Anspruch 1 | 10,0 mg |
| Milchzucker | 70,0 mg |
| Maisstärke | 50,0 mg |
| lösliche Stärke | 7,0 mg |
| Magnesiumstearat | 3,0 mg |
| | 140,0 mg |

Herstellung:

Wirkstoff und Magnesiumstearat werden mit einer wässrigen Lösung der löslichen Stärke granuliert, das Granalat getrocknet und innig mit Milchzucker und Maisstärke vermischt. Das Gemisch wird sodann zu Tabletten von 230 mg Gewicht verpreßt, die je 100 mg Wirkstoff enthalten.

c) Injektionslösungen

| | | |
|---|---|---|
| Wirkstoff gemäß Anspruch 1 | | 5,0 mg |
| Äthanolamin | | 60,0 mg |
| Natriumchlorid | | 20,0 mg |
| destilliertes Wasser | ad | 2 ml |

Herstellung:

Der Wirkstoff und die Hilfsstoffe werden in einer ausreichenden Menge destilliertem Wasser gelöst und mit der erforderlichen Menge Wasser auf die gewünschte Konzentration gebracht. Die Lösung wird filtriert und unter aseptischen Bedingungen in 2 ml Ampullen abgefüllt. Die Ampullen werden sterilisiert und verschlossen. Jede Ampulle enthält 5 mg Wirkstoff.

d) Kapseln

| | |
|---|---|
| Wirkstoff gemäß Anspruch 1 | 10,0 mg |
| Milchzucker | 250,0 mg |
| Maisstärke | 40,0 mg |
| Talk | 10,0 mg |
| | 310,0 mg |

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

e) Suppositorien

| | |
|---|---|
| Wirkstoff gemäß Anspruch 1 | 0,1 g |
| Kakaobutter (Fp. 36-37°C) | 1,6 g |
| Carnaubawachs | 0,1 g |
| | 1,8 g |

Kakaobutter und Carnaubawachs werden geschmolzen, gründlich vermengt und auf 45°C abgekühlt. In diese Masse wird der feinpulverisierte Wirkstoff eingerührt. Anschließend wird die Mischung in leicht vorgekühlten Suppositorienformen geeigneter Größe gegossen und abkühlen gelassen.

<u>Patentansprüche</u>

1. Aminosäurederivate der allgemeinen Formel

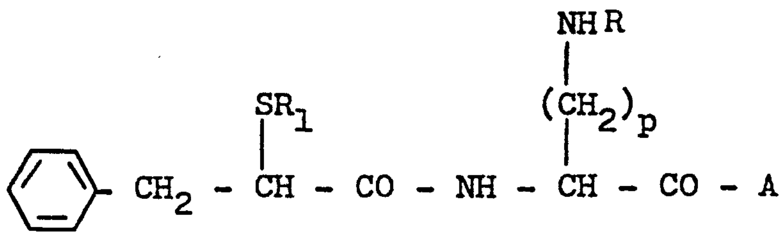

(I)

worin

R H, Acetyl, Benzoyl, Pivaloyl oder tert.-butoxycarbonyl;

$R_1$ H, Acetyl, Benzoyl, Pivaloyl oder den Rest

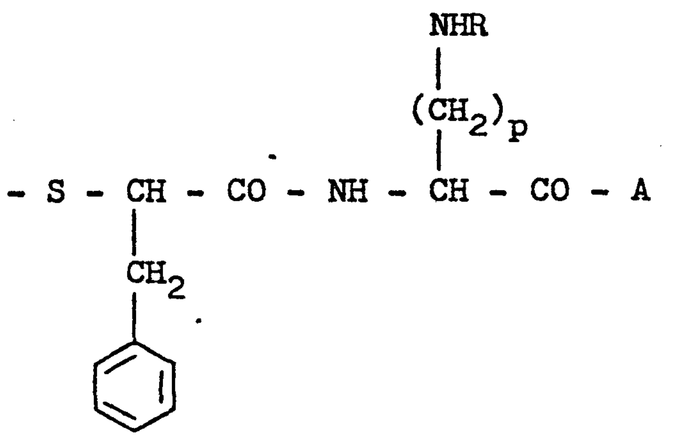

;

p eine der Zahlen 1 - 4;

A den Rest einer der α-Aminosäuren

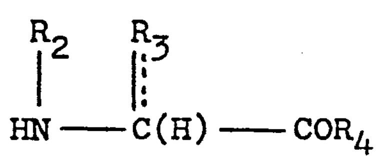

oder

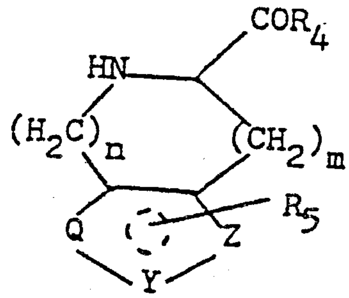

$R_2$ Wasserstoff, $C_1$-$C_4$-Alkyl, gerade oder verzweigt, $C_5$-$C_7$-Cycloalkyl, wobei ein Phenylring ankondensiert sein kann, Phenyl, Phenyl-$C_1$-$C_4$-alkyl, einen Hetero-$C_1$-$C_4$-alkylrest, wobei der Heterocyclus aus einem 5- oder 6-Ring mit 1 - 2 der Heteroatome O, S oder N besteht,

$R_3$ Wasserstoff, $C_1$-$C_4$-Alkyl, gerade oder verzweigt, Phenyl, Phenyl-$C_1$-$C_4$-alkyl oder einen Hetero-$C_1$-$C_4$-alkylrest, wobei der Heterocyclus aus einem 5- oder 6-Ring mit 1 - 2 der Heteroatome O, S oder N besteht, oder

$R_2$ und $R_3$ zusammen bilden mit dem N- und dem C-Atom einen 5-, 6- oder 7-gliedrigen Ring, der gesättigt sein oder eine Doppelbindung enthalten kann, oder einen 4-, 5- oder 6-gliedrigen Ring, der ein oder zwei weitere der Heteroatome O, S oder N enthält;

$R_4$ OH, $C_1$-$C_4$-ω-Hydroxyalkyl, $C_1$-$C_4$-Alkoxy, Phenyl-$C_1$-$C_4$-alkoxy, $C_1$-$C_4$-Alkylamino, $(C_1$-$C_4)_2$-Dialkylamino, eine der Gruppen

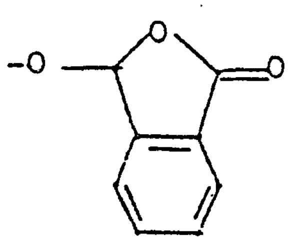 , 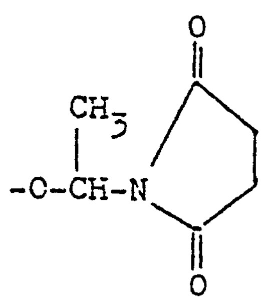 oder 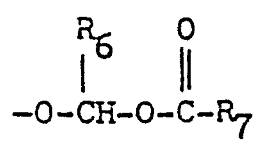

oder 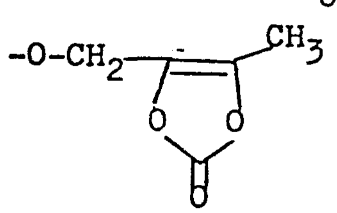

oder eine α-Aminosäure, die peptidartig mit der CO-Gruppe des Moleküls verknüpft ist;

$R_5$ $C_1$-$C_4$-Alkyl, $C_1$-$C_3$-Alkenyl, $C_1$-$C_3$-Alkinyl, gerade oder verzweigt, Hydroxy, Nitro, Amino, $C_1$-$C_4$-Alkoxy, Mercapto, $C_1$-$C_4$-Alkylthio, Hydroxy-$C_1$-$C_4$-alkyl, Mercapto-$C_1$-$C_4$-alkyl, F, Cl, Br, Amino-$C_1$-$C_4$-alkyl, Sulfonamido, Methylendioxy, Fluor-$C_1$-$C_4$-alkyl, Chlor-$C_1$-$C_4$-alkyl, Brom-$C_1$-$C_4$-alkyl, Cyano oder Trifluormethyl;

$R_6$ Wasserstoff oder Methyl;

$R_7$ $C_1$-$C_4$-Alkyl, gerade oder verzweigt, wobei der Alkylrest durch F, Cl, Br, $CF_3$, Phenyl oder Pyridyl substituiert sein kann;

Q, Y und Z O, S, $NR_8$, $CR_9$, $CHR_9$,

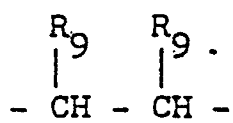 oder 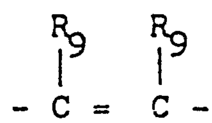 ,

mit der Maßgabe, daß nur einer der Reste X, Y und Z O, S

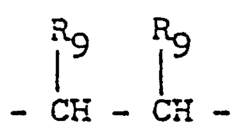 oder 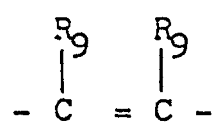 und

einer oder zwei der Reste X, Y und Z $NR_8$ bedeuten können;

$R_8$ Wasserstoff oder $C_1$-$C_4$-Alkyl, gerade oder verzweigt;

$R_9$ Wasserstoff, oder zusammen mit einem vicinal stehenden Rest $R_9$ einen Phenylring oder, für m und n = 1, dessen Dihydroform mit der Doppelbindung in Konjugation zur C-terminalen Carboxygruppe und

m und n jeweils 0,1 oder 2, wobei die Summe aus m und n 1 oder 2 ist, bedeuten

und deren Salze.

2. Aminosäurederivate nach Anspruch 1, dadurch gekennzeichnet, daß
   $R_1$ H,
   p = 4 und
   A Prolin oder Thiazolidin bedeutet
   und deren Salze.

3. N-[$N^{\alpha}$-(3-Phenyl-2-S-mercaptopropanoyl)-S-lysyl]-thiazolidin-4-S-carbonsäure und dessen Salze.

4. N-[$N^{\alpha}$-(3-Phenyl-2-S-mercaptopropanoyl)-S-lysyl]-S-prolin und dessen Salze.

5. Aminosäurederivate nach den Ansprüchen 1 - 4, dadurch gekennzeichnet, daß die asymmetrischen Kohlenstoffatome in S-Konfiguration vorliegen.

6. Verfahren zur Herstellung von Aminosäurederivaten der allgemeinen Formel I nach Anspruch 1, dadurch gekennzeichnet, daß man

   a) eine Säure der allgemeinen Formel

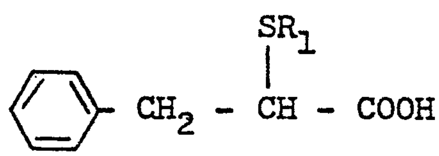

(II)

   worin
   $R_1$ die oben angegebene Bedeutung besitzt,
   mit einem Dipeptid der allgemeinen Formel

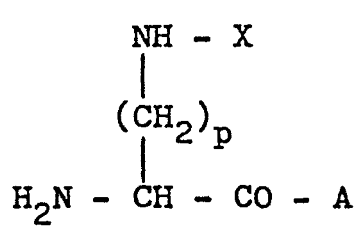

(III)

worin
A und p die oben angegebene Bedeutung besitzen und
X eine geeignete Schutzgruppe bedeutet, kondensiert,
oder daß man

b) eine Verbindung der allgemeinen Formel IIa

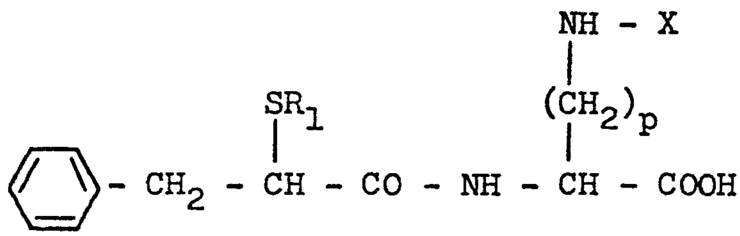

(IIa)

worin
$R_1$, X und p die oben angegebene Bedeutung haben,
mit einer Aminosäure A kondensiert, wobei A die
oben angegebene Bedeutung besitzt, oder daß man

c) eine Verbindung der allgemeinen Formel (V)

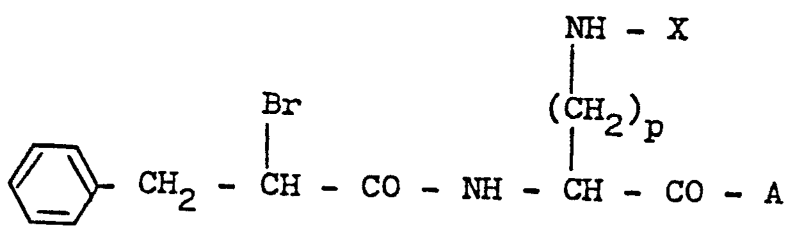

(V)

worin
X, p und A die oben angegebene Bedeutung besitzen,
mit einer Mercaptoverbindung der allgemeinen Formel

$$HS - R_1 \qquad (VI)$$

worin
$R_1$ die oben angeführte Bedeutung hat, umsetzt,
oder daß man

d) eine Verbindung der allgemeinen Formel

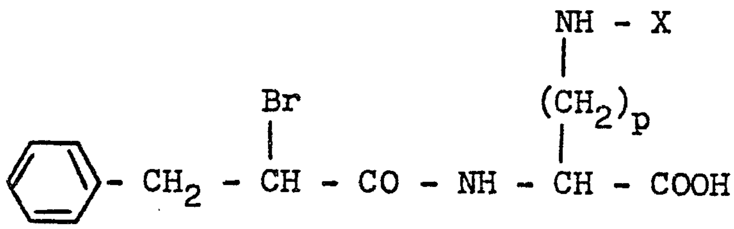

(IVa)

worin
X und p die oben angeführten Bedeutungen besitzen, mit einer Aminsäure A kondensiert, wobei A die vorstehend angegebene Bedeutung besitzt, und das so erhaltene Kondensationsprodukt der allgemeinen Formel V mit einer Mercaptoverbindung der allgemeinen Formel VI umsetzt,

e) eine Verbindung gemäß Formel I in welcher $R_1$ Acetyl, Benzoyl oder Pivaloyl bedeutet zur entsprechenden Verbindung gemäß Formel I in welcher $R_1$ Wasserstoff ist, umgewandelt wird

f) eine Verbindung gemäß Formel I in welcher $R_1$ Acetyl, Benzoyl oder Pivaloyl bedeutet und R Wasserstoff bedeutet, zur entsprechenden Verbindung gemäß Formel I in welcher $R_1$ Wasserstoff ist und R Acetyl, Benzoyl oder Pivaloyl ist, umgewandelt wird,und daß man etwa vorhandene Schutzgruppen einer geeigneten Stufe der Verfahren a), b), c), d), e) oder f) abgespalten werden,
und daß gegebenenfalls ein so erhaltenes Endprodukt der allgemeinen Formel I in ein Salz überführt.

7. Verfahren zur Herstellung dimerer Verbindungen der allgemeinen Formel I, worin $R_1$ die Gruppe

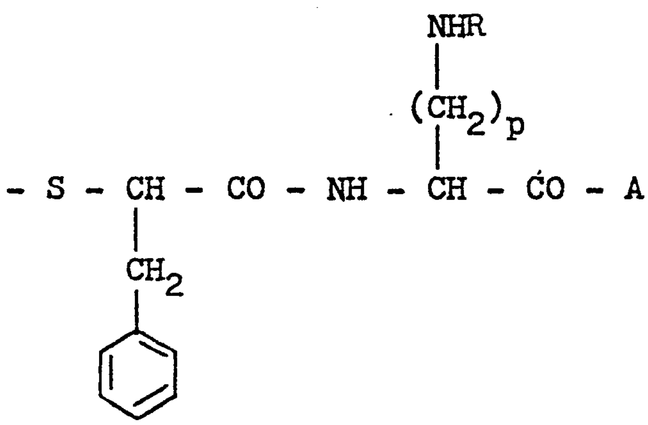

bedeutet und die übrigen Reste die in Anspruch 1 angegebene Bedeutung besitzen, dadurch gekennzeichnet, daß man eine Verbindung der allgemeinen Formel I, in der $R_1$ Wasserstoff bedeutet und die $NH_2$-Gruppe eine Schutzgruppe trägt, mit einem Oxydationsmittel behandelt, und daß man gegebenenfalls eine so erhaltene dimere Verbindung in ein Salz überführt.

8. Pharmazeutische Zubereitungen, enthaltend als Wirkstoff eine oder mehrere Verbindungen der Formel I oder deren Salze in Kombination mit üblichen Hilfs- und/oder Trägerstoffen.

9. Pharmazeutische Zubereitungen, enthaltend als Wirkstoff eine oder mehrere Verbindungen der allgemeinen Formel I in Gemisch mit anderen bekannten Saluretica beziehungsweise Diuretica und/oder Antihypertonica.

10. Verfahren zur Herstellung von pharmazeutischen Präparaten gemäß Anspruch 8, dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

11. Verwendung der Verbindungen nach Anspruch 1 als Hypotensiva.